# EUROPÄISCHE PATENTSCHRIFT

(11) **EP 0 419 584 B1**
(45) Veröffentlichungstag und Bekanntmachung des Hinweises auf die Patenterteilung: **12.07.1995**
(21) Anmeldenummer: 89911526.5
(22) Anmeldetag: 23.10.1989
(51) Int. Cl.: C07C 59/245, C07C 51/367, C07C 55/32, C07C 51/363, B01J 19/12

(54) **VERFAHREN ZUR HERSTELLUNG VON (+)ISOCITRONENSÄURE UND DEREN DERIVATE**
METHOD FOR PREPARING (+)-ISOCITRIC ACID AND ITS DERIVATIVES
PROCEDE POUR PREPARER DE L'ACIDE ISOCITRIQUE (+) ET SES DERIVES

(30) Priorität: 25.10.1988 DE 3836271
(43) Veröffentlichungstag der Anmeldung: 03.04.1991
(73) Patentinhaber: SCHMIDT, Günter, D-67125 Dannstadt-Schauernheim (DE)
(72) Erfinder: SCHMIDT, Günter, D-67125 Dannstadt-Schauernheim (DE)
(74) Vertreter: Fritsch, Klaus, Dipl.-Ing.
(86) Internationale Anmeldenummer: DE8900678
(87) Internationale Veröffentlichungsnummer: WO9004581

(56) Entgegenhaltungen:
- DE-C- 967 011
- GB-A- 600 993
- US-A- 4 272 442

## Beschreibung

Die Erfindung betrifft ein Verfahren zur Herstellung von (plus)-Isocitronensäure und deren Derivaten.

Isocitronensäure ist eine Substanz, die im Pharma- und Genußmittel bereich zunehmend an Bedeutung gewinnt und deren Bedarf daher steigt. Es ist weiterhin ein häufig verwendetes Säuerungsmittel, das aufgrund seiner Eigenschaften gut verträglich ist. In der Vergangenheit wurde Isocitronensäure durch Extraktionsverfahren aus Naturprodukten gewonnen, die sehr aufwendig waren, weswegen Isocitronensäure sehr teuer war und auch heute noch ist. Die chemische Darstellung von (+)-Isocitronensäure ist ebenfalls aufwendig und daher wurde zu biochemischen Verfahren übergegangen, in denen Isocitronensäure durch Fermentation von zucker- bzw. kohlenwasserstoffhaltigen Stoffen mit bestimmten Kulturen, z.B. Candida - sp. gewonnen wurde. Auch hier ist der Aufwand groß, wie z.B. aus der DE-AS 20 46 576 hervorgeht, in der ein Verfahren zur Abtrennung von Citronensäure aus einem Gemisch mit Isocitronensäure beschrieben wird.

Aufgabe der Erfindung ist es, ein Verfahren der eingangs genannten Art anzugeben, mit dem (+) - Isocitronensäure einfach und preisgünstig herstellbar ist.

Diese Aufgabe wird erfindungsgemäß gelöst durch ein Verfahren gemäß dem kennzeichnenden Merkmalen des Anspruches 1.

Es ist dabei überraschend festgestellt worden, daß die Ausgangsstoffe - Malein- und/oder Fumarsäure und/oder deren Derivate und Halogenessigsäure und deren Derivate dann am besten miteinander reagieren, wenn sie ultraviolettem Licht einer bestimmten Wellenlänge ausgesetzt sind, d.h. fotomechnischer Energie (UV-Licht), deren Wellenlänge im Bereich zwischen 200 bis 300 nm liegt. Dabei hat es sich als besonders vorteilhaft herausgestellt, wenn die Lichtenergie besonders hoch ist; mit einfachen Worten kann gesagt werden, daß die Ausbeute desto höher bei verkürzter Zeitdauer umso höher liegen, je höher die zugeführte Lichtenergie ist. Wichtig ist auch, daß die zur Reaktion erforderliche Temperatur lediglich durch Lichteinwirkung erreicht werden kann. Die Temperaturbereiche, bei denen die Maleinsäure und/oder Fumarsäure und/oder deren Derivate mit Halogenessigsäure und deren Derivate miteinander reagieren, beträgt erfindungsgemäß zwischen ca. 60 bis 120 Grad Celsius.

Der besondere Vorteil des erfindungsgemäßen Verfahrens liegt im wesentlichen darin, daß die (+) - Isocitronensäure auf einfache Weise leicht durch Bestrahlung zweier oder mehrerer Ausgangsstoffe hergestellt werden kann.

Die Herstellung von (+) - Isocitionensäure kann diskontinuierlich in einem Behälter oder kontinuierlich in einem Durchlaufverfahren durchgeführt werden, indem die Ausgangsstoffe durch ein für ultraviolettes Licht durchlässiges Rohrsystem hindurchgeleitet werden, an dessen Außenseite eine geeignete Anzahl das richtige Licht abgebender Lampen vorgesehen sind. Zum Beispiel können hierfür Systeme benutzt werden, die zur Entkeimung von Wasser dienen.

Anhand von Ausführungsbeispielen soll die Erfindung näher erläutert und beschrieben werden:

### Beispiel 1

Es werden 98g Maleinsäureanhydrid (1,0 Mol) mit 94g Chloressigsäure (1 Mol) vermischt und bei 80 Grad C unter Rühren 30 Stunden lang mit UV-Licht einer Wellenlänge von 254 nm bestrahlt. Das Reaktionsprodukt wird bei 80 Grad mit 300 ml Wasser gelöst und mit Natronlauge (NaOH-Lösung) auf einen pH - Wert 10 bis 11 gebracht. Weiterhin wird es 60 Minuten am Rückfluss gekocht, auf Raumtemperatur abgekühlt und über einen Kationenaustauscher bis zum Na-Durchbruch gegeben. Das Eluat wird mit KOH auf einen pH-Wert von 3,5 eingestellt und bei niedriger Temperatur soweit eingeengt, bis Trübung eintritt. Bei niedriger Temperatur wird das Eluat mehrere Stunden ruhen lassen; die Kristallmasse wird danach abgetrennt und getrocknet. Die Ausbaute ist Kalium-Isocitrat von 156,9 g, was 68,26% des theoretisch errechneten, erzielbaren Wertes darstellt.

### Beispiel 2

98g Maleinsäureanhydrid werden mit 94,5g Essigsäure gemischt und auf 120 Grad erwärmt. Die klare Schmelze wird mit ultraviolettem Licht bestrahlt, indem in die klare Schmelze eine UV-Lampe eingetaucht wird. Das Gemisch wird 16 Stunden bei 120 Grad C unter Rühren bestrahlt.

Das Reaktionsprodukt wird wie bei Beispiel 1 weiterbehandelt und aufgearbeitet.

Die Ausbeute beträgt 161,92g Kaliumisocitrat; dies entspricht 70,4 % des theoretisch errechneten Wertes.

### Beispiel 3

114g Maleinsauredibutylester (0,5 Mol) werden mit 75g Chloressigsäurebutylester (o,5 Mol) gemischt, auf 80 Grad C erwärmt und 18 Stunden mit ultraviolettem Lichtmit einer Wellenlänge von 254 nm bestrahlt. Das Reaktionsprodukt wird mit einem berechneten Überschuß an Natriumlauge versetzt und das entstehende Butanol abdistiliert.

Die Aufarbeitung erfolgt wie in Beispiel 1. Die Ausbeute beträgt 75,1g Kaliumisocitrat, was 65,3 % des theoretisch errechneten Wertes darstellt.

### Beispiel 4

86,1g Fumarsäurediethylester (o,5 Mol) werden mit 61,3g Chloressigsäurethylester (o,5 Mol) vermischt und bei 80 Grad unter Rühren 18 Stunden mit UV-Licht einer Wellenlänge von 254 nm bestrahlt.

Die Aufarbeitung des Reaktionsproduktes erfolgt wie im Beispiel 1 bzw. im Beispiel 3.

Die Ausbeute beträgt 81,65g Kaliumisocitrat; dies entspricht 71% des theoretisch errechneten Wertes.

### Beispiel 5

58,0g Fumarsäure (o,5 Mol) werden mit 47,3g Cloressigsäure (o,5 Mol) in 250g Dioxan gelöst. In die Lösung wird eine ultraviolettes Licht einer Wellenlänge von 254 nm abgebende Lampe eingetaucht und 14 Stunden unter Rühren bei 80 Grad Celsius bestrahlt. Das Reaktionsgemisch wird mit 400g Wasser verdünnt und mit Natronlauge auf einen pH-Wert von 10 bis 11 gebracht und 1,5 Stunden am Rückfluss gekocht. Nach Abkühlen auf Raumtemperatur wird die Lösung über einen Kationenaustauscher gegeben. Das kationenfreie Eluat wird mit KOH auf einen pH-Wert von 3,5 eingestellt, auf 50 %Volumen bei niedriger Temperatur eingeengt und abgekühlt. Die Kristallmasse wird abgetrennt, mit 80%igem ETOH gewaschen und getrocknet. Die Ausbeute beträgt 63,2g Kaliumisozytrat, was 54,9 % des theoretisch errechneten Wertes beträgt.

Die Formeldarstellung ist wie folgt:
Ausgangsprodukt: Maleinsäureanhydrid 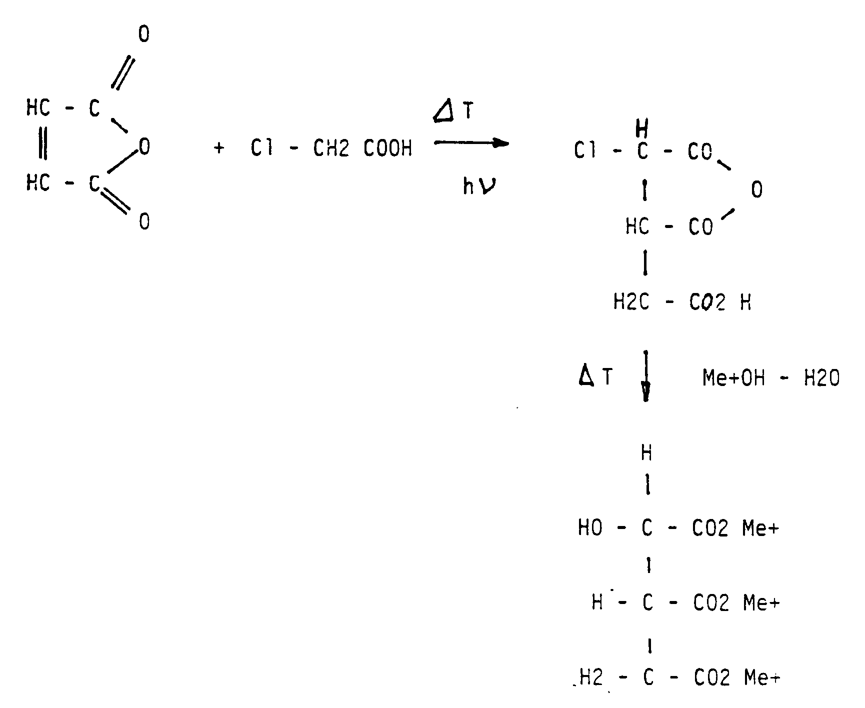
Hierin sind die Metallionen Me+ Ka+ - und/oder Na+ - Ionen.

Ausgangsstoff: Fumarsäure bzw. -ester 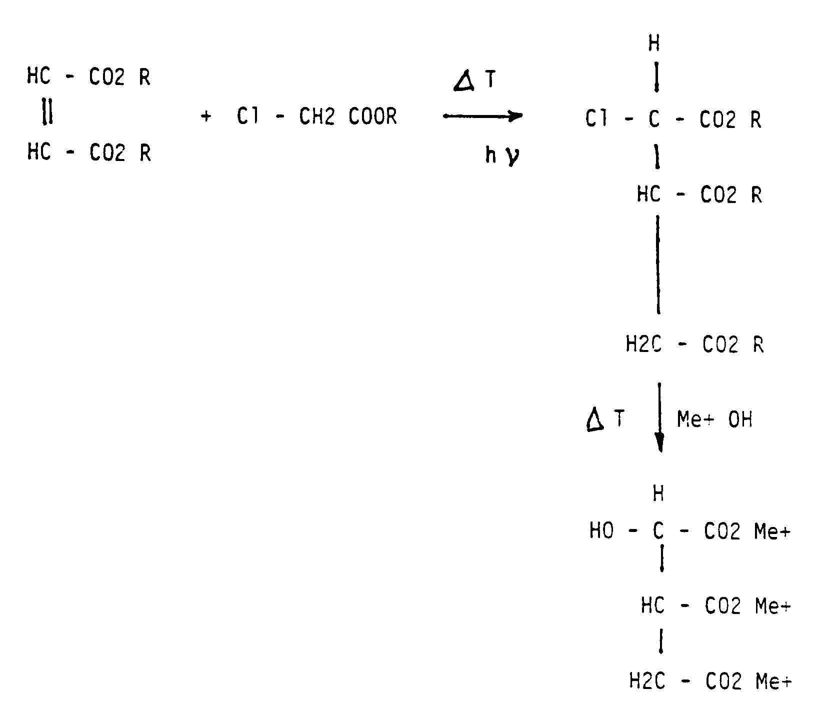
Hierin sind Me+ Metallionen z.B. Na+ - und/oder Ka+ - Ionen. R ist ein Alkylrest, z.B. CH2 CH2 CH2 CH3 Butyl -, wie Beispiel 3, oder - CH2 CH3 Ethyl, wie Beispiel 4.

In der beigefügten Zeichnung sollen Ausführungsbeispiele von Herstellungsanordnungen zur Durchführung des Verfahrens näher erläutert und beschrieben werden.

Es zeigt:
- Fig. 1: eine schematische Darstellung einer Vorrichtung zur diskontinuierlichen Herstellung von (+) Isocitronensäure und
- Fig. 2: eine Anordnung zur kontinuierlichen Herstellung von Isocitronensäure.

In einem Behälter 10 befindet sich eine Heizflüssigkeit 11, z.B. Wasser oder Öl, die mittels einer Heizung 12 auf die für die Durchführung des Verfahrens erforderliche Temperatur zwischen 60 bis 120 Grad gebracht wird. In die Flüssigkeit eingetaucht ist ein Reaktionsbehälter 13, in dem sich ein Rührer 14 sowie eine darin eingefügte UV-Lampe 15 befindet. Der Rührer 14 wird über eine Rührwelle 16 von einem Elektromotor 17 angetrieben; an der Oberseite des Reaktionsbehälters 13 schließt ein Ablaßrohr 18 an, durch den evtl. bei der Reaktion entstehende gasförmige Bestandteile abgeführt und gegebenenfalls abdestilliert werden können. Insbesondere bei der Verwendung von Estern wird der abdestillierende Teil in Form von Alkohol, Butanol oder anderen Lösungsmitteln wieder zurückgewonnen, insbesondere zur Vermeidung von Geruchsbelästigungen; allerdings ist ein solcher Kühler nicht zwingend erforderlich.

Die Leistung der ultravioletten Lampe ist nicht von wesentlicher Bedeutung; man stellt aber fest, daß mit Erhöhung der Lampenleistung die Reaktion schneller und damit besser abläuft.

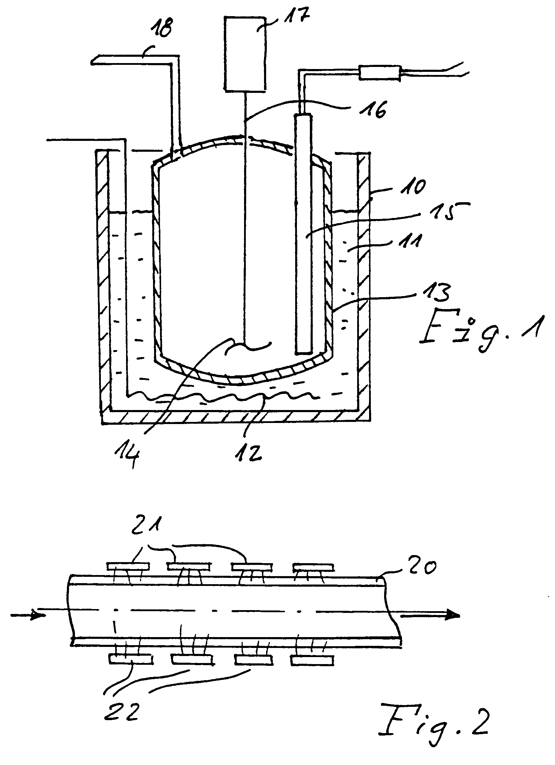

Die Fig. 2 zeigt eine weitere Ausführung, mit der die Isocitronensäure kontinuierlich hergestellt werden kann. Durch ein für ultraviolettes Licht durchlässiges Rohr 20 wird das für die Herstellung von Isocitronensäure vorgesehene Ausgangsgemisch hindurchgeleitet und um das Rohr 20 herum sind in entsprechenden Abständen ultraviolettes Licht abgebende Lampen 21,22 angeordnet. Die Ausführung ist ähnlich wie bei Sterilisationseinrichtungen für Abwässer unter Verwendung von ultraviolettem Licht. Aufgrund der UV-Bestrahlung über die Lampen 21 und 22 heizt sich das durch das Rohr 20 hindurchfließende Gemisch selbst auf, so daß weitere eigenständige Heizeinrichtungen nicht erforderlich sein müssen. Die Ausgangsprodukte, die bei der Ausführung nach Fig. 2 auftreten, können dann in üblicher Weise , siehe oben angegebene Beispiele 1 bis 5 , weiter aufgearbeitet werden.

In den Bespielen ist angegeben, daß die Wellenlänge 254 nm betragen hat. Dies ist mehr oder weniger zufällig; es könnte auch Licht einer anderen Wellenlänge eingesetzt werden.

## Patentansprüche

1. Verfahren zur Herstellung von (+)-Isocitronensäure und deren Derivaten, **dadurch gekennzeichnet,** daß Maleinsäure und/oder Fumarsäure und/oder deren Derivate durch fotomechanische Energie Halogenessigsäure und deren Derivate bei erhöhter Temperatur addieren und daß danach die Halogengruppe gegen eine Hydroxylgruppe ausgetauscht wird.

2. Verfahren nach Anspruch 1, dadurch gekennzeichnet, daß die fotomechanische Energie ultraviolettes Licht mit einer Wellenlänge von ca. 200 bis 300 nm ist.

3. Verfahren nach Anspruch 1 und 2, dadurch gekennzeichnet, daß die Temperatur, bei der die Maleinsäure und/oder Fumarsäure und/oder deren Derivate mit Halogenessigsäure und deren Derivate miteinander reagieren, ca. 60 bis 120 Grad beträgt.

## Claims

1. A process for the preparation of (+)-Isocitric acid and its derivates, characterized in that maleic acid and /or fumaric acid and /or their derivates under to effect of UV radiation and elevated temperature add halogenated acetic acid and/or its derivates and that subsequently the halogen group will be replaced with a hydroxyl group.

2. The process according to claim 1, characterized in that the UV radiation of the wave length range from 200 to 300 nanometers.

3. The process according to claim 1 and 2, characterized in that the temperature, at which the reaction between maleic acid and/or fumaric acid and/or their derivatives with halogenated acetic acid and /or its derivatives takes place, is the range from ca. 60 to 120 °C.

## Revendications

1. Procédé de fabrication de l'acide (+) iso-citrique et de ses dérivés, caractérisé par le fait que, l'acide malique et/ou l'acide fumarique et/ou leurs dérivés additionnent à température élevée avec l'acide acétique halogéné et ses dérivés sous l'effet de l'énergie photomécanique et qu'ensuite le groupe halogène est échangé contre un groupe hydroxyle.

2. Procédé selon revendication 1, caractérisé par le fait que, l'énérgie photomécanique est apportée par une lumière ultra-violette de longueur d'onde de 200 à 300 nm.

3. Procédé selon revendications 1 et 2, caractérisé par le fait que, la température de réaction de l'acide maléique et/ou de l'acide fumarique et/ou de leurs dérivés avec l'acide acétique halogéné et ses dérivés se situe entre 60 et 120 degrés.
